# EUROPÄISCHE PATENTSCHRIFT

(11) **EP 0 895 460 B1**
(45) Veröffentlichungstag und Bekanntmachung des Hinweises auf die Patenterteilung: **09.01.2002**
(21) Anmeldenummer: 97922863.2
(22) Anmeldetag: 25.04.1997
(51) Int. Cl.: A61B 17/34

(54) **TROKARDORN MIT EINER SPITZE**
TROCAR SPIKE WITH A POINT
TROCART MUNI D'UNE POINTE

(30) Priorität: 25.04.1996 DE 19616609
(43) Veröffentlichungstag der Anmeldung: 10.02.1999
(73) Patentinhaber: Karl Storz GmbH & Co. KG, 78532 Tuttlingen (DE)
(72) Erfinder: MANHES, Hubert, F-03200 Vichy (FR)
(74) Vertreter: Münich, Wilhelm, Dr.
(86) Internationale Anmeldenummer: DE9700838
(87) Internationale Veröffentlichungsnummer: WO9740758

(56) Entgegenhaltungen:
- EP-A- 0 512 759
- WO-A-96/01132
- WO-A-96/32148
- DE-A- 2 835 812
- DE-U- 9 109 909

## Beschreibung

### Technisches Gebiet

Die Erfindung bezieht sich auf einen Trokardorn mit einer Spitze, die ein Durchdringen z.B. der Bauchdecke und damit ein Einsetzen in die Bauchhöhle ermöglicht.

Derartige Trokardorne, die manchmal auch nur als Trokare bezeichnet werden, dienen beispielsweise dazu, einen für die Laparoskopie geeigneten künstlichen Zugang durch die Bauchdecke in die Bauchhöhle zu schaffen.

### Stand der Technik

Bei laparoskopischen Eingriffen wird der Trokardorn in einen Trokarschaft, der manchmal auch als Trokarhülse oder auch nur als Trokar bezeichnet wird, eingesetzt. Der Trokardorn mit dem ihm umgebenden Trokarschaft wird durch die Bauchdecke gestoßen. Nach erfolgtem Einsetzvorgang wird der Trokardorn aus dem Trokarschaft herausgezogen, so daß in den Kanal des Trokarschafts andere Instrumente, wie beispielsweise ein Endoskop, eine Zange, eine Schere und/oder dergleichen eingesetzt werden können.

Bei bestimmten Operationstechniken wird vor dem Einsetzen des Trokars in die Bauchdecke eine spezielle Nadel eingeführt, mittels der in die Bauchhöhle ein Gas, wie insbesondere CO₂ insuffliert wird.

Eine derartige Nadel ist beispielsweise die als "Veressnadel" bekannte Nadel, die normalerweise einen Durchmesser von 2,4 mm aufweist. Nach dem "Aufblasen" der Bauchhöhle mit Insufflationsgas, d.h. nach der Erzeugung eines Überdrucks von bis zu 6650 Pascal(50 Torr), wird durch einen anderen "Einstich" oder nach dem Zurückziehen der Veressnadel durch denselben Einstich der Trokardorn mit Trokarschaft eingeführt. Der Trokardorn hat bei laparoskopischen Eingriffen typischerweise einen Durchmesser von 10 mm.

Beim Einführen des Trokardorns kann es zu ungewollten Verletzungen des Bauchraums kommen; weiterhin kann es vorkommen, daß die Spitze des Trokardorns nicht in die eigentliche Bauchhöhle eingesetzt wird.

In der US-PS 5,407,427, von der bei der Formulierung des Oberbegriffs des Anspruchs 1 ausgegangen wird, ist ein Trokardorn mit einer Spitze beschrieben, die ein Durchdringen der Bauchdecke ermöglicht. In dem Trokardorn ist ein in Richtung der Längsachse des Trokardorns verlaufender Insufflationskanal vorgesehen, dessen Achse und damit dessen distale Austrittsöffnung von der Spitze einen Abstand in Richtung senkrecht zur Längsachse des im übrigen rotationssymmetrisch aufgebauten Dorns hat. Die Austrittsöffnung des Insufflationskanals liegt neben der Dornspitze.

Dieser bekannte Trokardorn ermöglicht es, auf die Verwendung einer Verres-Nadel zu verzichten (s. Spalte 9, Z. 51 ff. der US-PS 5,407,427).

Nachteilig ist jedoch, daß es beim Durchstoßen der Bauchdecke vorkommen kann, daß sich die Austrittsöffnung mit Gewebe "zusetzt", so daß eine Insufflation beispielsweise des Bauchraums nicht bzw. nur in unzureichendem Umfange mehr möglich ist.

### Darstellung der Erfindung

Der Erfindung liegt die Aufgabe zugrunde, einen Trokardorn gemäß dem Oberbegriff des Patentanspruchs 1 derart weiterzubilden, daß es bei weiterhin möglichem Verzicht auf eine Verres-Nadel beim Einsetzen des Trokars nicht zu Komplikationen insbesondere dadurch kommen kann, daß sich der Insufflationskanal im Trokardorn zusetzt. Er soll weiter gewährleisten, daß es nicht zu Komplikationen aufgrund eines falschen Einsetzvorgangs oder aufgrund von Verletzungen durch die Insufflaionsnadel kommen und daß unmittelbar nach dem Durchstoßen der Bauchdecke ohne jede Störung und ohne zusätzlichen Arbeitsgang mit der Insufflation begonnen werden kann.
Es soll ein Instrument geschaffen werden, das eine Insufflationsnadel, wie beispielsweise eine Verresnadel mit einem Trokardorn in ein und demselben Instrument verbindet, wodurch es möglich wird, einen direkten Zugang zur Bauchhöhle zu schaffen, so daß die Vorfälle bzw. Komplikationen, wie sie beim getrennten Einsatz der Instrumente auftreten können, verhindert werden.

Eine erfindungsgemäße Lösung dieser Aufgabe ist im Patentanspruch 1 angegeben. Weiterbildungen der Erfindung sind Gegenstand der Ansprüche 2 ff.

Erfindungsgemäß ist in dem Insufflationskanal verschiebbar eine Insufflationsnadel angeordnet, die eine seitlich angeordnete Austrittsöffnung für das Insufflationsfluid aufweist.

Mit dem erfindungsgemäßen Trokardorn kann in gewohnter Weise ein direkter Zugang beispielsweise zur Bauchhöhle geschaffen werden, ohne daß es zu Komplikationen aufgrund eines falschen Einsatzvorgangs oder Aufgrund von Verletzungen durch die Insufflationsnadel kommen kann. Durch die seitliche Anordnung der Austrittsöffnung für das Insufflationsfluid und insbesondere des Gases an der Insufflationsnadel wird zuverlässig verhindert, daß sich die Austrittsöffnung und damit der Insufflationszugang beim Durchstoßen der Bauchdecke mit Gewebe zusetzt. Damit ist gewährleistet, daß unmittelbar nach dem Durchstoßen der Bauchdecke ohne jede Störung mit der Insufflation begonnen werden kann.

Bei einer Weiterbildung ist das distale Ende der Insufflationsnadel stumpf. Aufgrund der stumpfen Ausbildung des distalen Endes der Insufflationsnadel ist es nicht möglich, daß eine an der Insufflationsnadel vorhandene Spitze nach dem Durchstoßen der Bauchdecke Verletzungen im Bauchraum hervorruft.

In jedem Falle ist durch die versetzte Anordnung der Spitze und des Kanals der Trokardorn weiterhin in der gewohnten Weise einsetzbar.

Mit dem erfindungsgemäßen Instrument, das eine Insufflationsnadel, wie beispielsweise eine Verresnadel mit einem Trokardorn in ein und demselben Instrument verbindet, ist es möglich, einen direkten Zugang zur Bauchhöhle zu schaffen, so daß die Vorfälle bzw. Komplikationen, wie sie beim getrennten Einsatz der Instrumente auftreten können, zumindest vermindert, wenn nicht sogar vollständig vermieden werden.

Bei der im Anspruch 2 gekennzeichneten Weiterbildung steht das distale Ende der Insufflationsnadel im Normalzustand über die Spitze des Trokardorns vor. Da die Insufflationsnadel gegen eine elastische Kraft verschiebbar derart gelagert ist, daß sie hinter die Spitze zurück verschiebbar ist, wird die Insufflationsnadel beim Durchstoßen der Bauchwand hinter die Eingriffsfläche des Dorns zurückgeschoben. Hierdurch wird nicht nur ein leichtes Durchdringen der Bauchwand gewährleistet, sondern auch die Nadel vor Beschädigungen etc. geschützt.

Der Trokardorn kann dabei die Außenform eines an sich bekannten Trokardorns haben und insbesondere eine konisch abgeschrägte Spitze aufweisen. Die Spitze kann bei einer weiteren bevorzugten Ausführungsform wenigstens zwei Abschrägungen aufweisen, die Schneidkanten bilden. Dabei können die Austrittsöffnungen des Insufflationskanals in einer der Abschrägungen vorgesehen sein.

Aufgrund der erfindungsgemäßen Kombination einer Verres-nadel und eines Trokars in einem Instrument ist es weiterhin möglich, daß der Durchmesser des Insufflationskanals und damit der Insufflationsnadel größer als das Kaliber herkömmlicher Verresnadeln ist. Insbesondere kann der Durchmesser ca. 3 mm und mehr betragen.

Dennoch ist es möglich, im Trokardorn zusätzlich (wenigstens) einen Spülkanal vorzusehen, mit dem ein Spülfluid, wie eine Flüssigkeit eingelassen und/oder abgepumpt werden kann.

Der erfindungsgemäße Trokardorn kann selbstverständlich als Einweg-Trokar ausgeführt werden. Bevorzugt ist er jedoch für die Mehrfachverwendung sterilisierbar und insbesondere für die Durchführung des Steriliservorgangs zerlegbar ausgebildet. Durch die Zerlegbarkeit ist sichergestellt, daß auch Hohlräume etc. sicher sterilisiert werden.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen hinsichtlich der Offenbarung aller im Text nicht näher erläuterten erfindungsgemäßen Einzelheiten ausdrücklich verwiesen wird.

Es zeigen:
- Fig. 1: eine Aufsicht auf einen erfindungsgemäßen Trokardorn,
- Fig. 2: eine Seitenansicht, und
- Fig. 3: eine Ansicht von unten des in Figur 1 dargestellten Trokardorns.

### Darstellung von Ausführungsbeispielen

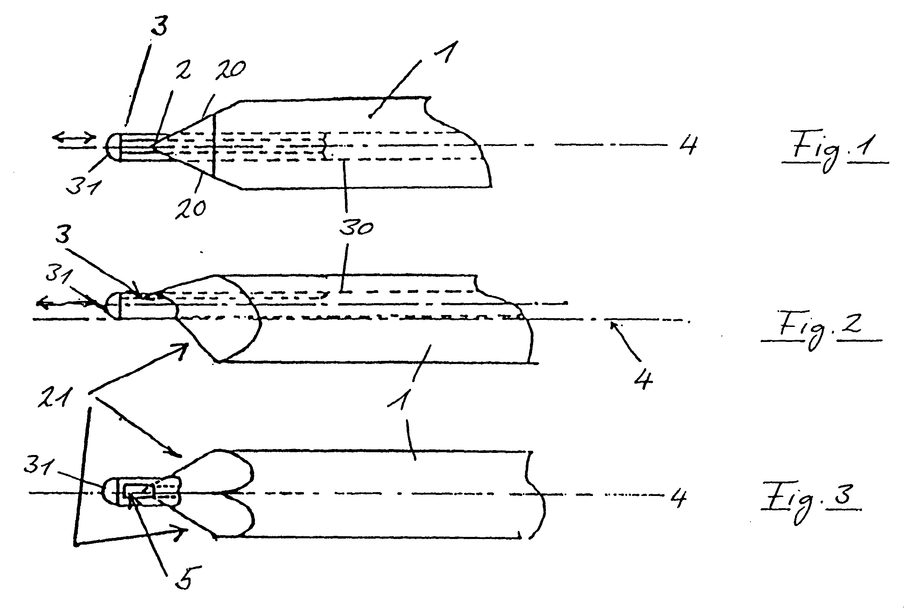

In den Figuren 1 bis 3 ist ein erfindungsgemäßer Trokardorn 1 in verschiedenen Ansichten dargestellt.

Der Trokardorn 1 weist eine konische abgeschrägte Spitze 2 auf. Die Spitze 2 wird bei dem dargestellten Ausführungsbeispiel durch zwei Abschrägungen 20 gebildet, die die Schneidkanten 21 bilden.

Bei dem erfindungsgemäßen Instrument ist ein Insufflationskanal 30 vorgesehen, dessen Längsachse gegenüber der Achse, die durch die Spitze des Trokardorns geht, versetzt ist, also einen Abstand senkrecht zu den jeweiligen Achsen aufweist.

Bei dem gezeigten Ausführungsbeispiel sind ferner sowohl die Spitze 2 als auch der Insufflationskanal 30 außer axial zur Mittelachse 4 des Trokardorns angeordnet, so daß der Trokardorn keine rotationssymmetrische Grundform hat. Dies ist in der in Fig. 2 dargestellten Seitenansicht deutlich zu erkennen. Hierdurch werden - ohne Beeinträchtigung des Durchstoßens der Bauchdecke - die Effizienz der Insufflation erhöht sowie die Anordnung des Insufflationskanals sowie weitere Kanäle erleichtert. Insbesondere ist es möglich, den Insufflationskanal in der geometrischen Mittelachse des Trokardorns anzuordnen.

Der Insufflationskanal 30 mündet distalseitig in einer Ausnehmung bzw. einer Austrittsöffnung im kegelförmigen (distalen) Teil des Trokardorns 1. Bei einer bevorzugten Ausführungsform ist der Insufflationskanal 30 ein durchgehender Kanal, so daß in ihm von der proximalen Seite her ohne Zerlegen des Instruments eine "Insufflationsnadel" 3 eingesetzt werden kann, die insbesondere nach dem Verresprinzip ausgestaltet sein kann.

Eine elastische Kraft, die beispielsweise von einer Feder aufgebracht wird, spannt die Insufflationsnadel 3 derart vor, daß sie über die Fläche der Abschrägung, in der sich die Austrittsöffnung und insbesondere auch über die Spitze 2 des Trokardorns 1 in Richtung der Achse 4 vorsteht. Durch eine von außen wirkende Kraft, wie sie beispielsweise beim Durchbohren der Bauchdecke erzeugt wird, wird die Insufflationsnadel 3 hinter die Fläche der entsprechenden Abschrägung zurückgeschoben. Dies ist durch einen Doppelpfeil in der Figur 1 und 2 angedeutet.

Ferner ist das distale Ende 31 der Nadel 3 stumpf ausgebildet. Damit kann die Insufflationsnadel weder im vorgeschobenen "Grundzustand" noch im zurückgezogenen "Einführ-Zustand" Schädigungen bei Organen, Blutgefäßen etc. hervorrufen.

In der Nadel 3 ist ein gestrichelt dargestellter Kanal-der eigentliche Insufflationskanal - vorgesehen, durch den z.B. CO₂ vom proximalen Ende des Instruments zum distalen Ende geleitet werden kann. Dieser Kanal mündet in einer Öffnung 5, durch den das Insufflationsgas austritt. Erfindungsgemäß ist die Öffnung 5 nicht an der Stirnseite der Nadel 3, sondern an einer Seitenfläche der Insufflationsnadel 3 vorgesehen. Aufgrund dieser Anordnung ist die Öffnung 5 geschützt und kann beim Durchstoßen bzw. Durchbohren der Bauchdecke nicht mit Gewebeteilen etc. zugesetzt werden.

Das Kaliber der Nadel 3 ist bevorzugt größer als das Kaliber von Standardnadel, wie beispielsweise herkömmliche Verresnadeln.

Ferner kann die Möglichkeit zur Spülung und Sterilisierung bestehen. Hierzu kann wenigstens ein weiterer - nicht dargestellter - Kanal vorgesehen sein, durch den ein Spülfluid zugeführt oder abgesaugt wird. Zuführung und Absaugung können auch alternierend erfolgen.

Bei dem gezeigten Ausführungsbeispiel hat der Trokar (bzw. der Trokardorn) 1 einen Durchmesser von 10 mm. Die Insufflationsnadel 3 weist einen Durchmesser von 3 mm auf.

Das bereits beschriebene und in den Figuren nicht dargestellte Federsystem ermöglicht das Zurückziehen der Nadel 3 hinter die Abschrägung 20, sobald sich der Trokardorn 1 zunächst mit der stumpfen Spitze 31 der Nadel 30 und dann mit der entsprechenden Abschrägung 20 auf der Bauchwand abstützt. Damit wird die Spitze 2 des Trokardorns 1 freigegeben, die unter dem vom Operateur ausgeübten Druck die Bauchwand durchsticht. Sobald die Bauchwand durchstochen ist, kehrt die Insufflationsnadel 3 wieder in ihre vorgeschobene Stellung zurück, die in den Figuren dargestellt ist.

In diesem Zustand erfolgt die Insufflation. Dabei ist die Trokarspitze 2 von der Nadel 3 "bedeckt". Das stumpfe Ende der Nadel 3 stellt damit einen Schutz vor Verletzungen des Bauchraums durch die Spitze 2 dar.

Da die Spitze der Insufflationsnadel "stumpf", also nicht zugespitzt ist, wird jegliche Aggressivität, d.h. jegliche Gefährdung in der Bauchhöhle vermieden.

Bei einer typischen laparoskopischen Operation wird der Nabeleinschnitt in der Tiefe linear in der Stellung von 6 bis 12 Uhr gelegt. Die Bauchwand wird unter kräftigem Erfassen der Haut beiderseits des Nabels und unter diesm in der Weise angehoben, daß ein Winkel von etwa 45° gegenüber der Horizontalen entsteht.

Der Trokardorn wird mit dem Insufflationsgerät verbunden, das mit geringer Leistung, d.h. typischerweise 1 bis 2 Liter Gas pro min. betrieben wird.

Wenn der Trokardorn 1 in einem in der Zeichnung nicht dargestellten Trokarschaft eingeführt worden ist, wird er in den Nabelschnitt eingeführt und eingeschoben.

Die stumpfe Spitze der Nadel 3 zieht sich zurück, die Spitze 2 an der Abschrägung des Trokardorns 1 durchquert die Sehnenplatte der geraden Muskeln und anschließend das Bauchfell, dessen geringerer Widerstand es möglich macht, daß der stumpfe Dorn seine Schutzfunktion erfüllen kann.

Gleichzeitig strömt das Gas - beispielsweise CO₂ - in die Bauchhöhle ein und hebt das virtuelle Vakuum zwischen den beiden serösen Häuten auf: es entsteht eine "Luftblase" zur Sicherheit, die jegliches Einbrechen der darunterliegenden Strukturen verhindert.

Der Fortschritt des Vorgangs kann an der Druckmeßeinrichtung des Insufflationsgeräts verfolgt werden:

Auf anfänglichen Überdruck folgt ein Unterdruck, sobald sich das stumpfe distale Ende 31 der Nadel 3 in der Peritonealhöhle befindet.

Damit ist es für den Operateur möglich, mit hoher Sicherheit den Fortschritt des Einführvorgangs des Trokardorns mit umgebender Trokarhülse zu erkennen.

Anschließend wird der Trokardorn 1 um 1 bis 2cm weiter eingeschoben, so daß der Schaft korrekt eingeschoben ist. Nun kann die eigentliche Endoskopie beginnen.

Mit dem erfindungsgemäßen Trokardorn, der auch als Direkttrokar oder als "Pneumotrokar" bezeichnet wird, wird die Möglichkeit realisiert, die Insufflationsfunktion einer Veressnadel mit der Trokarfunktion in ein und demselben Trokarinstrument in einem Handgriff zusammenzufassen. Hierdurch ist gerade in Bezug auf die scharfe und spitze Trokarspitze eine erhöhte Sicherheit für den Patienten gewährleistet.

Die erfindungsgemäße Ausbildung gewährleistet insbesondere einen hervorragenden Schutz vor Gefäßverletzung oder anderen Verletzungen infolge der Gefährlichkeit der Spitze nach dem Durchtritt durch die Bauchwand. Der erfindungsgemäße Trokardorn hat weiterhin den Vorteil, daß zwei Arbeitsvorgänge bzw. Handbewegungen zu einem einzigen Vorgang kombiniert sind; ferner ist die schnelle Ausführbarkeit des Einführvorgangs und die vereinfachte Kontrolle über die Druckmeßeinrichtung am Insufflationsgerät von Vorteil. Darüberhinaus werden Komblikationen deutlich verringert, sofern der Trokar nicht bei einem vernarbten Abdomen eingesetzt wird.

Vorstehend ist die Erfindung anhand eines Ausführungsbeispiels ohne Beschränkung der allgemeinen Ausführbarkeit beschrieben worden. Insbesondere kann der erfindungsgemäße Trokar auch für andere Operationen als für laparoskopische Eingriffe eingesetzt werden. Gegebenenfalls kann auch durch den Kanal in der Nadel eine Flüssigkeit anstelle eines Fluids eingeleitet werden.

Die seitliche Anordnung der Gas-Austrittsöffnung 5 hat den Vorteil, daß die Austrittsöffnung beim Durchstoßen von Gewebe geschützt ist. Gleichzeitig ist im zurückgezogenen Zustand der Gasfluß praktisch unterbunden. Beim Ausfahren der Insufflationsnadel, das nach dem Durchstoßen beispielsweise der Bauchdecke insbesondere durch Federkraft erfolgen kann, beginnt automatisch die Insufflation, ohne daß der Chirurg den Insufflationsvorgang gesondert stoppen müßte.

## Patentansprüche

1. Trokardorn (1) mit einer Spitze (2), die ein Durchdringen z.B. der Bauchdecke ermöglicht, und mit einem in Richtung der Längsachse des Trokardorns (1) verlaufenden Insufflationskanal (30), der sowohl neben der geometrischen Mittelachse (4) des Trokardorns (1) als auch neben der Trokardornspitze liegt,
**dadurch gekennzeichnet, daß** in dem Insufflationskanal (30) verschiebbar eine Insufflationsnadel (3) angeordnet ist, die einen sich darin befindlichen Kanal und eine seitlich angeordnete Öffnung (5) für das Insufflationsfluid aufweist und
daß die Spitze (2) des Trokardorns (1) außerhalb der geometrischen Mittelachse (4) des Trokardorns (1) liegt und mindestens zwei Abschrägungen (20) aufweist, die Schneidkanten (21) bilden.

2. Trokardorn (1) nach Anspruch 1,
**dadurch gekennzeichnet, daß** die Insufflationsnadel (3) gegen eine elastische Kraft verschiebbar derart gelagert ist, daß ihr distales Ende (31) im nicht durch äußere Kräfte beaufschlagten Zustand über die Spitze (2) des Trokardorns (1) vorsteht, und daß sie durch äußere Kräfte hinter die Spitze (2) zurück verschiebbar ist.

3. Trokardorn (1) nach Anspruch 1 oder 2,
**dadurch gekennzeichnet, daß** das distale Ende der Insufflationsnadel (3) stumpf ist.

4. Trokardorn (1) nach Anspruch 3,
**dadurch gekennzeichnet, daß** sich das distale Ende (31) der Insufflationsnadel (3) im zurückgeschobenen Zustand in die Oberfläche des Trokardorns (1) einfügt.

5. Trokardorn (1) nach Anspruch 1 bis 4,
**dadurch gekennzeichnet, daß** die Austrittsöffnung des Insufflationskanals (30) in einer der Abschrägungen (20) vorgesehen ist.

6. Trokardorn (1) nach einem der Ansprüche 1 bis 4,
**dadurch gekennzeichnet, daß** der Durchmesser des Insufflationskanals (30) und damit der Insufflationsnadel (3) 3 mm und mehr betragen kann.

7. Trokardorn (1) nach Anspruch 6,
**dadurch gekennzeichnet, daß** die Insufflationsnadel (3) einen Durchmesser von 3 mm besitzt.

8. Trokardorn (1) nach einem der Ansprüche 1 bis 7,
**dadurch gekennzeichnet, daß** zusätzlich wenigstens ein Spülkanal im Trokardorn (1) vorgesehen ist.

9. Trokardorn (1) nach einem der Ansprüche 1 bis 8,
**dadurch gekennzeichnet, daß** der Trokardorn (1) sterilisierbar und insbesondere hierzu zerlegbar ist.

10. Trokardorn (1) nach einem der Ansprüche 1 bis 9,
**dadurch gekennzeichnet, daß** der Insufflationskanal (30) die Spitze (2) nicht durchsetzt.

## Claims

1. Trocar spike (1) with a point (2) permitting the penetration of the abdominal wall, for instance, and comprising an insufflation duct (30) extending in the direction of the longitudinal axis of the trocar spike (1), which duct is positioned both beside the geometric centre axis (4) of said trocar spike (1) and beside said trocar spike point,
**characterised in that** an insufflation needle (3) is disposed for sliding in said insufflation duct (30), which needle includes a passage formed therein and a laterally disposed opening (5) for the insufflation fluid, and
that said point (2) of said trocar spike (1) is positioned outside the geometric centre axis (4) of the trocar spike (1) and presents at least two bevels (20) forming cutting edges (12).

2. Trocar spike (1) according to Claim 1,
**characterised in that** said insufflation needle (3) is supported for sliding in opposition against a resilient force such that, in the state without application of external forces, its distal end (31) projects beyond the point (2) of the trocar spike, and that it is adapted to be pushed back behind the point (2) by external forces.

3. Trocar spike (1) according to Claim 1 or 2,
**characterised in that** the distal end of said insufflation needle (3) is blunt.

4. Trocar spike (1) according to Claim 3,
**characterised in that**, in the pushed-back state, the distal end (31) of said insufflation needle (3) fits snugly into the surface of the trocar spike (1).

5. Trocar spike (1) according to the Claims 1 to 4,
**characterised in that** the discharge opening of said insufflation duct (30) is provided in one of said bevels (20).

6. Trocar spike (1) according to any of the Claims 1 to 4,
**characterised in that** the diameter of said insufflation duct (30) and hence of said insufflation needle (3) may correspond to 3 mm and more.

7. Trocar spike (1) according to Claim 6,
**characterised in that** said insufflation needle (3) presents a diameter of 3 mm.

8. Trocar spike (1) according to any of the Claims 1 to 7,
**characterised in that** additionally at least one irrigation duct is provided in the trocar spike (1).

9. Trocar spike (1) according to any of the Claims 1 to 8,
**characterised in that** the trocar spike (1) is adapted to be sterilised and dismountable to this end in particular.

10. Trocar spike (1) according to any of the Claims 1 to 9,
**characterised in that** said insufflation duct (30) does not pass through said point (2).

## Revendications

1. Goujon de trocart (1) muni d'une pointe (2), qui permet la pénétration de la paroi abdominale, par exemple, comprenant un canal d'insufflation (30), qui s'étend en direction de l'axe longitudinal du goujon de trocart (1), ce canal étant positionné non seulement à côté de l'axe central géométrique (4) du goujon de trocart (1), mais aussi à côté de la pointe du goujon de trocart,
**caractérisé en ce qu'**une aiguille d'insufflation (3) est disposée de façon à glisser dans ledit canal d'insufflation (30), cette aiguille renfermant un conduit formé à son intérieur, et un orifice (5) disposée latéralement pour le fluide d'insufflation, et
**en ce que** ladite pointe (2) dudit goujon de trocart (1) est positionnée en dehors de l'axe central géométrique (4) du goujon de trocart (1), en présentant au moins deux biseaux (20), qui forment des arrêtes coupantes (12).

2. Goujon de trocart (1) selon la revendication 1,
**caractérisé en ce que** ladite aiguille d'insufflation (3) est appuyée de façon à glisser à l'encontre d'une force élastique, d'une manière que, en l'état sans application des forces extérieures, l'extrémité distale (31) fasse saillie de la pointe (2) du goujon de trocart, et qu'elle soit apte à être poussée derrière la pointe (2) par des forces extérieures.

3. Goujon de trocart (1) selon la revendication 1 ou 2,
**caractérisé en ce que** l'extrémité distale de ladite aiguille d'insufflation (3) est non pointue.

4. Goujon de trocart (1) selon la revendication 3,
**caractérisé en ce qu'**en état poussé en retour, l'extrémité distale (31) de ladite aiguille d'insufflation (3) s'emboîte bien dans la surface du goujon de trocart (1).

5. Goujon de trocart (1) selon les revendications 1 à 4,
**caractérisé en ce que** l'orifice de sortie dudit canal d'insufflation (30) est formé dans un desdits biseaux (20).

6. Goujon de trocart (1) selon une quelconque des revendications 1 à 4,
**caractérisé en ce que** le diamètre dudit canal d'insufflation (30) et ainsi de ladite aiguille d'insufflation (3) peut correspondre à 3 mm et plus.

7. Goujon de trocart (1) selon la revendication 6,
**caractérisé en ce que** ladite aiguille d'insufflation (3) a un diamètre de 3 mm.

8. Goujon de trocart (1) selon une quelconque des revendications 1 à 7,
**caractérisé en ce qu'**au moins un canal d'irrigation supplémentaire est formé dans le goujon de trocart (1).

9. Goujon de trocart (1) selon une quelconque des revendications 1 à 8,
**caractérisé en ce que** le goujon de trocart (1) est apte à être stérilisé, en étant démontable, en particulier, à cette fin.

10. Goujon de trocart (1) selon une quelconque des revendications 1 à 9,
**caractérisé en ce que** ledit canal d'insufflation (30) ne passe pas à travers ladite pointe (2).
